# EUROPÄISCHE PATENTANMELDUNG

(11) **EP 0 759 425 A2**
(43) Veröffentlichungstag der Anmeldung: **26.02.1997**
(21) Anmeldenummer: 96112606.7
(22) Anmeldetag: 05.08.1996
(51) Int. Cl.: C07C 209/58, C07C 231/02, C07C 51/36, C07B 53/00

(54) **Verfahren zur Herstellung von optisch aktiven 1-Aryl-alkylaminen**

(30) Priorität: 17.08.1995 DE 19530205
(71) Anmelder: BAYER AG, 51368 Leverkusen (DE)
(72) Erfinder: Laue, Christian, Dr., 40789 Monheim (DE); Stelzer, Uwe, Dr., 51399 Burscheid (DE); Lantzsch, Reinhard, Dr., 42115 Wuppertal (DE)

(57) **Zusammenfassung**

Nach einem neuen Verfahren lassen sich optisch aktive 1-Aryl-alkylamine der Formel 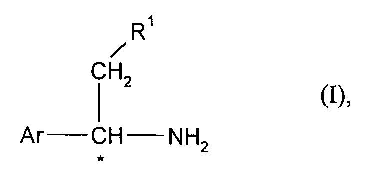 in welcher
R¹ und Ar die in der Beschreibung angegebenen Bedeutungen haben,
herstellen, indem man
a) 2-Aryl-propensäuren der Formel 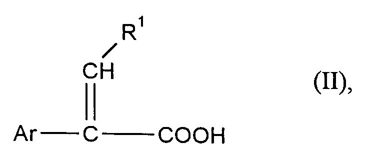 mit Wasserstoff in Gegenwart eines chiralen Komplexes aus einem Übergangsmetallsalz und einem chiralen Bisphosphin, sowie in Gegenwart eines Verdünnungsmittels und gegebenenfalls in Gegenwart eines tertiären Amins umsetzt,
b) die dabei entstehenden optisch aktiven 2-Aryl-propansäuren der Formel 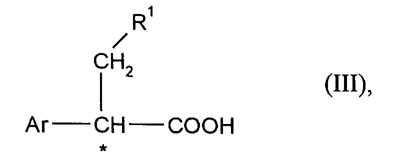 zunächst mit Thionylchlorid, gegebenenfalls in Gegenwart eines Verdünnungsmittels, und dann mit Ammoniak, gegebenenfalls in Gegenwart eines Verdünnungsmittels und gegebenenfalls in Gegenwart eines Katalysators, umsetzt und
c) die dabei erhaltenen optisch aktiven 2-Aryl-propansäureamide der Formel 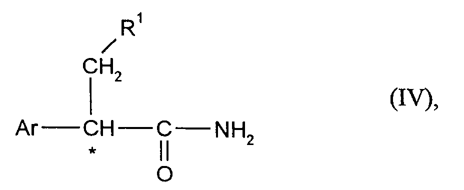 mit Natriumhypochlorit oder Natriumhypobromit in Gegenwart von Wasser umsetzt.

## Beschreibung

Die vorliegende Erfindung betrifft ein neues Verfahren zur Herstellung von optisch aktiven 1-Aryl-alkylaminen, die als Zwischenprodukte zur Synthese von Wirkstoffen mit fungiziden Eigenschaften verwendet werden können.

Es ist bereits bekannt, daß sich optisch aktive Amine herstellen lassen, indem mar Racemate von Aminen mit 2-(4-Hydroxyphenoxy)-propionsäure umsetzt, aus dem entstehenden Diastereomeren-Gemisch das gewünschte Produkt abtrennt und daraus durch Behandlung mit Base das optisch aktive Amin freisetzt (vgl. JP 07-010 813). Ungünstig an diesem Verfahren ist, daß nur jeweils maximal die Hälfte des eingesetzten Racemates in das angestrebte optisch aktive Amin überführt werden kann. Der verbleibende, nicht benötigte Antipode muß racemisiert und einer erneuten Racematspaltung unterworfen werden.

Weiterhin ist schon bekannt, daß man (R)-1-(4-Chlor-phenyl)-ethylamin erhält, wenn man racemisches 1-(4-Chlor-phenyl)-ethylamin in Gegenwart von Ethanol mit (S)-(-)-N-Phenylcarbamat-milchsäure umsetzt, den dabei entstehenden Kristallbrei absaugt und mit wäßriger Natronlauge in Gegenwart von Methylenchlorid behandelt (vgl. EP-A 0 341 475). Nachteilig an diesem Verfahren ist jedoch, daß ein beachtlicher Anteil an (S)-(-)-N-Phenylcarbamat-milchsäure in der nach dem Absaugen des Kristallbreis anfallenden Mutterlauge vorhanden ist und daraus nur in aufwendiger Weise isoliert werden kann.

Wie ferner in der DE-A 4 332 738 offenbart wird, lassen sich optisch aktive primäre und sekundäre Amine herstellen, indem man zunächst racemisches Amin mit bestimmten Estern in Gegenwart einer Hydrolase enantioselektiv acyliert, dann das Gemisch aus optisch aktivem Amin und optisch aktivem acylierten Amin trennt und dadurch ein Enantiomer des Amins erhält und gegebenenfalls aus dem optisch aktiven acylierten Amin durch Amidspaltung das andere Enantiomer erhält. Auch die Wirtschaftlichkeit dieses Verfahrens wird dadurch beeinträchtigt, daß nur ein Teil des eingesetzten Racemates ausgenutzt wird. Das übrige Produkt muß erst racemisiert werden, bevor es wieder in den Prozeß zurückgeführt werden kann. Außerdem handelt es sich bei dem eingesetzten Enzym um eine relativ teure Komponente.

Aus J. Chem. Soc. Commun. 1993, 1831-1832 ist zu entnehmen, daß sich Racemate von Estern mit Hilfe von Ammoniak in Gegenwart bestimmter Enzyme, wie Candida antarctica Lipase SP 435, enantioselektiv in optisch aktive Amide überführen lassen. Auch dieses Verfahren ist mit dem Nachteil behaftet, daß das gewünschte Produkt in aufwendiger Weise abgetrennt und die nicht benötigte Komponente racemisiert werden muß.

Schließlich geht aus J. Org. Chem. 52, 3174-3176 (1987) hervor, daß sich 2-Arylpropensäuren in Gegenwart von Rutheniumkomplexen, die chirale Bisphosphin-Liganden enthalten, stereospezifisch hydrieren lassen. Eine enantioselektive Umsetzung der anfallenden optisch aktiven Säuren zu Aminen wird allerdings nicht mitgeteilt.

Es wurde nun gefunden, daß man optisch aktive 1-Aryl-alkylamine der Formel 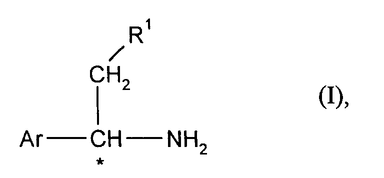 in welcher
- R¹: für Wasserstoff oder Alkyl steht und
- Ar: für Phenyl oder Naphthyl steht, wobei jeder dieser Reste einfach bis fünffach, gleichartig oder verschieden substituiert sein kann durch Halogen, Alkyl, Alkoxy, Halogenalkyl, Halogenalkoxy, Cyano, Dialkylamino, Phenyl und/oder Benzyl,
erhält, indem man
a) 2-Aryl-propensäuren der Formel 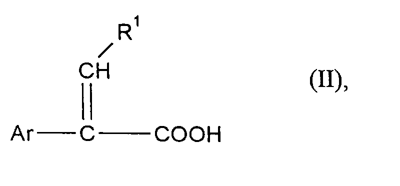 in welcher
   R¹ und Ar die oben angegebenen Bedeutungen haben,
   mit Wasserstoff in Gegenwart eines chiralen Komplexes aus einem Übergangsmetallsalz und einem chiralen Bisphosphin, sowie in Gegenwart eines Verdünnungsmittels und gegebenenfalls in Gegenwart eines tertiären Amins umsetzt,
b) die dabei entstehenden optisch aktiven 2-Aryl-propansäuren der Formel 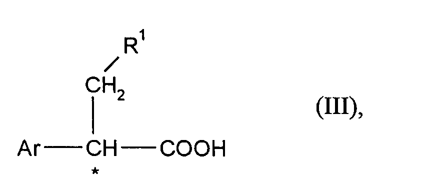 in welcher
   Ar und R¹ die oben angegebenen Bedeutungen haben,
   zunächst mit Thionylchlorid, gegebenenfalls in Gegenwart eines Verdünnungsmittels, und dann mit Ammoniak, gegebenenfalls in Gegenwart eines Verdünnungsmittels und gegebenenfalls in Gegenwart eines Katalysators, umsetzt und
c) die dabei erhaltenen optisch aktiven 2-Aryl-propansäureamide der Formel 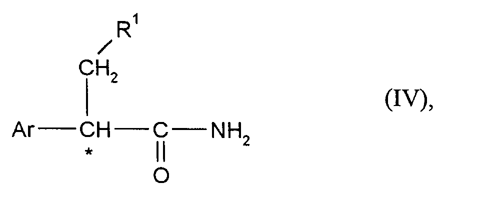 in welcher
   R¹ und Ar die oben angegebenen Bedeutungen haben,
   mit Natriumhypohalogeniten der Formel

   Na-O-Hal (V),

   in welcher
   Hal für Chlor oder Brom steht,
   in Gegenwart von Wasser umsetzt.

Es ist als äußerst überraschend zu bezeichnen, daß sich optisch aktive 1-Aryl-alkylamine nach dem erfindungsgemäßen Verfahren in hoher Ausbeute und sehr guter optischer Reinheit herstellen lassen. Aufgrund des bekannten Standes der Technik konnte nämlich nicht damit gerechnet werden, daß die mehrstufige Synthese enantioselektiv und ohne nennenswerte Nebenreaktionen abläuft.

Das erfindungsgemäße Verfahren weist eine Reihe von Vorteilen auf. So ermöglicht es die Herstellung einer Vielzahl von optisch aktiven 1-Aryl-alkylaminen in hoher Ausbeute und sehr guter optischer Reinheit. Günstig ist es auch, daß sowohl die Ausgangsstoffe als auch die Reaktionskomponenten in einfacher Weise zugänglich sind und auch in größeren Mengen zur Verfügung stehen. Von besonderem Vorteil ist schließlich, daß keine unerwünschten Enantiomeren anfallen, die für weitere Umsetzungen nicht direkt verwertbar sind.

Setzt man 2-(4-Chlor-phenyl)-propensäure in Gegenwart eines Ruthenium-(II)-Komplexes von (5,5'-Dichloro-6,6'-dimethoxy-biphenyl-2,2'-diyl)-bis-diphenylphosphin (= ClMeOBIPHEP) als Katalysator mit Wasserstoff unter Druck um, und läßt das dabei entstehende Produkt zunächst mit Thionylchlorid und dann mit Ammoniak reagieren und behandelt man das so erhaltene Produkt mit Natriumhypobromit, so kann der Verlauf des erfindungsgemäßen Verfahrens durch das folgende Formelschema veranschaulicht werden.

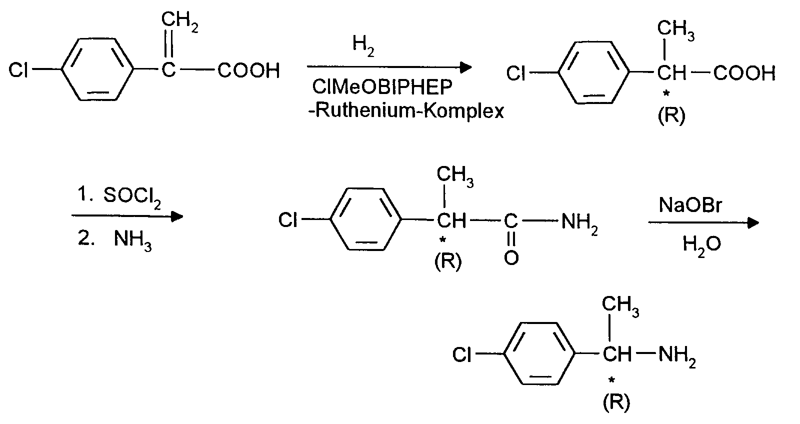

In dem obigen Reaktionsschema sind die asymmetrisch substituierten Kohlenstoffatome der optisch aktiven Verbindungen jeweils durch (*) gekennzeichnet. In gleicher Weise sind die Chiralitätszentren auch in anderen Formeln markiert.

Die bei der Durchführung des erfindungsgemäßen Verfahrens als Ausgangsstoffe benötigten 2-Aryl-propensäuren sind durch die Formel (II) allgemein definiert. In dieser Formel steht
- R¹: vorzugsweise für Wasserstoff oder geradkettiges oder verzweigtes Alkyl mit 1 bis 6 Kohlenstoffatomen, und
- Ar: steht vorzugsweise für Phenyl oder Naphthyl, wobei jeder dieser Reste einfach bis dreifach, gleichartig oder verschieden substituiert sein kann durch Fluor, Chlor, Brom, geradkettiges oder verzweigtes Alkyl mit 1 bis 4 Kohlenstoffatomen, geradkettiges oder verzweigtes Alkoxy mit 1 bis 4 Kohlenstoffatomen, Halogenalkyl mit 1 bis 4 Kohlenstoffatomen und 1 bis 5 gleichen oder verschiedenen Halogenatomen, Halogenalkoxy mit 1 bis 4 Kohlenstoffatomen und 1 bis 5 gleichen oder verschiedenen Halogenatomen, Cyano, Dialkylamino mit 1 bis 4 Kohlenstoffatomen in jeder Alkylgruppe, Phenyl und/oder Benzyl.

Besonders bevorzugt sind Verbindungen der Formel (II), in denen
- R¹: für Wasserstoff, Methyl, Ethyl, n-Propyl, Isopropyl, n-Butyl, iso-Butyl, sek.-Butyl oder tert.-Butyl steht und
- Ar: für Phenyl oder Naphthyl steht, wobei jeder dieser beiden Reste einfach bis dreifach, gleichartig oder verschieden substituiert sein kann durch Fluor, Chlor, Brom, Methyl, Ethyl, n-Propyl, Isopropyl, n-Butyl, iso-Butyl, sek.-Butyl, tert.-Butyl, Methoxy, Ethoxy, Trichlormethyl, Trifluormethyl, Difluormethyl, Trifluormethoxy, Difluorchlormethoxy, Difluormethoxy, Cyano, Dimethylamino, Diethylamino, Phenyl und/oder Benzyl.

Die 2-Aryl-propensäuren der Formel (II) sind bekannt oder lassen sich nach bekannten Methoden herstellen (vgl. J. Org. Chem. 52, 3174-3176 (1987), Tetrahedron Lett. 1977, (52), 4631-4634 und EP-A 0 529 444).

Als Katalysatoren kommen bei der Durchführung der ersten Stufe des erfindungsgemäßen Verfahrens alle üblichen, zur enantioselektiven Hydrierung verwendeten Übergangsmetall-(II)-Komplexe mit chiralen Bisphosphinen als Liganden in Frage. Bevorzugt sind derartige Komplexe, die Rhodium, Ruthenium oder Iridium als Übergangsmetall enthalten.

Als Beispiele für besonders bevorzugte Ruthenium-Komplexe seien genannt:

Ru₂Cl₄B₂(S) (VI),

[Ru Hal' Q B]^{⊕}Y^{⊖} (VII),

Ru Bₙ OOCR²OOCR³ (VIII),

[Ru Hₓ Bₙ]^{m⊕} Yₘ^{⊖} (IX),

[Ru Hal' (PR⁴₂R⁵)B]⁽²⁺⁾ Hal'₂⊖ (X),

[Ru H Hal' B₂] (XI),

[B Ru (acac)₂] (XII),

[B Ru Y₂] (XIII),

worin
- acac: für Acetylacetonat steht,
- B: für ein chirales Bisphosphin steht und
- Hal': für Halogen, insbesondere für Iod, Chlor oder Brom steht,
- R² und R³: gleich oder verschieden sind und für Alkyl mit bis zu 9 C-Atomen, vorzugsweise bis zu 4 C-Atomen stehen, welches gegebenenfalls substituiert ist durch Halogen, insbesondere Fluor, Chlor oder Brom, oder für Phenyl stehen, welches gegebenenfalls durch Alkyl mit 1 bis 4 C-Atomen substituiert ist, oder für eine α-Aminoalkylsäure mit vorzugsweise bis zu 4 C-Atomen stehen,
oder
gemeinsam für eine Alkylidengruppe mit bis zu 4 C-Atomen stehen,
- R⁴ und R⁵: jeweils gleich oder verschieden sind und für gegebenenfalls substituiertes Phenyl stehen, vorzugsweise substituiert durch Alkyl mit 1 bis 4 C-Atomen oder Halogen,
- Y: für Cl, Br, J, ClO₄, BF₄, CH₃-COO- oder PF₆ steht,
- Q: für einen unsubstituierten oder substituierten Benzolring wie p-Cymol steht,
- S: für ein tertiäres Amin, wie z.B. Triethylamin, Tri-n-Butylamin oder Pyridin steht,
- n und m: jeweils für 1 oder 2 stehen,
- x: für 0 oder 1 steht,
wobei in Formel (IX) ein n für 1 und m für 2 steht, wenn x = 0 bedeutet, und n für 2 und m für 1 steht, wenn x = 1 bedeutet.

Ganz besonders bevorzugt sind Ruthenium-Komplexe, die Bisphosphine der folgenden Formeln als Liganden enthalten: 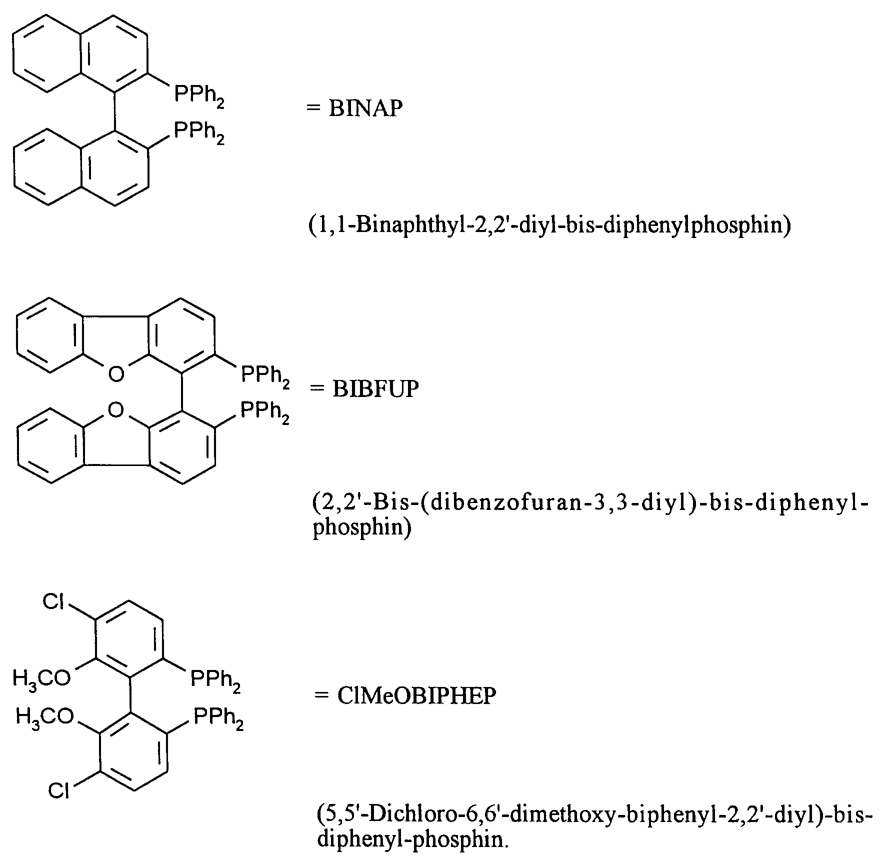 Ph = Phenyl

Beispiele für besonders geeignete Ruthenium-Katalysatoren sind:
BINAP Ru (OAc)₂
[(ClMeOBIPHEP)₂ Ru₂ Cl₄]NEt₃
[BINAP Ru cym I]I
[ClMeOBIPHEP Ru cym I] I
[BIBFUP Ru cym I] I
[BINAP₂ Ru₂ Cl₄] NEt₃
[BIBFUP₂ Ru₂ Cl] NEt₃
   cym = 4-Isopropyltoluol

Die erfindungsgemäß verwendbaren Katalysatoren aus Übergangsmetallsalz und chiralem Bisphosphin sind bekannt oder lassen sich nach bekannten Verfahren herstellen (vgl. J. Org. Chem. 52, 3174-3176 (1987), EP-A 0 529 444 und DE-A 4 330 730).

Als tertiäre Amine kommen bei der Durchführung der ersten Stufe des erfindungsgemäßen Verfahrens alle üblichen aliphatischen, alicyclischen und aromatischen Amine in Betracht. Vorzugsweise verwendbar sind Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Als Verdünnungsmittel kommen bei der Durchführung der ersten Stufe des erfindungsgemäßen Verfahrens alle üblichen inerten organischen Solventien in Frage. Vorzugsweise verwendbar sind gegebenenfalls halogenierte aliphatische, alicyclische und aromatische Kohlenwasserstoffe, wie Hexan, Cyclohexan, Benzol, Toluol, Xylol, Methylenchlorid, Chloroform und Chlorbenzol, ferner Alkohole, wie Methanol und Ethanol, und außerdem Ether, wie Tetrahydrofuran und Dioxan, sowie auch Gemische der genannten Solventien.

Die Reaktionstemperaturen können bei der Durchführung der ersten Stufe des erfindungsgemäßen Verfahrens innerhalb eines größeren Bereiches variiert werden. Im allgemeinen arbeitet man bei Temperaturen zwischen 5°C und 100°C, vorzugsweise zwischen 10°C und 70°C.

Die Umsetzung in der ersten Stufe des erfindungsgemäßen Verfahrens wird unter erhöhtem Druck durchgeführt. Im allgemeinen arbeitet man unter einem Druck zwischen 1 und 200 bar, vorzugsweise zwischen 10 und 100 bar.

Bei der Durchführung der ersten Stufe des erfindungsgemäßen Verfahrens setzt man auf 1 mol an 2-Aryl-propensäure der Formel (II) im allgemeinen 0,00005 bis 0,05 mol, vorzugsweise 0,0005 bis 0,01 mol, an chiralem Katalysator-Komplex aus Übergangsmetallsalz und chiralem Bisphosphin sowie einen Überschuß an Wasserstoff und gegebenenfalls 0,1 bis 1,1 mol an tertiärem Amin ein. Die Aufarbeitung erfolgt nach üblichen Methoden. Im allgemeinen geht man so vor, daß man das Reaktionsgemisch unter vermindertem Druck einengt, den verbleibenden Rückstand in einem mit Wasser wenig mischbaren organischen Lösungsmittel aufnimmt, dann nacheinander mit verdünnter, wäßriger Salzsäure, mit wäßriger Natriumhydrogencarbonat-Lösung und schließlich mit wäßriger Kochsalz-Lösung ausschüttelt und danach die organische Phase trocknet und unter vermindertem Druck einengt.

Als Verdünnungsmittel kommen bei der Umsetzung der 2-Aryl-propansäuren der Formel (III) mit Thionylchlorid in der zweiten Stufe des erfindungsgemäßen Verfahrens alle für derartige Reaktionen üblichen organischen Solventien in Betracht. Vorzugsweise verwendbar sind halogenierte aliphatische Kohlenwasserstoffe, wie Methylenchlorid, Chloroform und Tetrachlorkohlenstoff. Es ist aber auch möglich, in Abwesenheit von Verdünnungsmitteln zu arbeiten.

Die Reaktionstemperaturen können bei der Durchführung der Chlorierung in der zweiten Stufe des erfindungsgemäßen Verfahrens innerhalb eines bestimmten Bereiches variiert werden. Im allgemeinen arbeitet man bei Temperaturen zwischen -10°C und 100°C, vorzugsweise zwischen 0°C und 80°C.

Bei der Umsetzung der 2-Aryl-propansäuren mit Thionylchlorid in der zweiten Stufe des erfindungsgemäßen Verfahrens arbeitet man im allgemeinen unter Atmosphärendruck. Es ist aber auch möglich, unter etwas vermindertem Druck zu arbeiten.

Bei der Durchführung der zweiten Stufe des erfindungsgemäßen Verfahrens setzt man auf 1 mol an optisch aktiver 2-Aryl-propansäure im allgemeinen 1 bis 5 mol, vorzugsweise 1,1 bis 2 mol an Thionylchlorid ein. Die Aufarbeitung erfolgt nach üblichen Methoden. Im allgemeinen geht man so vor, daß man das Reaktionsgemisch nach beendeter Umsetzung unter vermindertem Druck einengt. Das erhaltene Produkt kann ohne zusätzliche Reinigung für die weitere Synthese verwendet werden.

Zur Überführung der optisch aktiven 2-Aryl-propansäurechloride in Amide können in der zweiten Stufe des erfindungsgemäßen Verfahrens sowohl Ammoniak als auch wäßrige Ammoniak-Lösung eingesetzt werden.

Als Verdünnungsmittel kommen bei der Herstellung der optisch aktiven 2-Aryl-propansäureamide der Formel (IV) in der zweiten Stufe des erfindungsgemäßen Verfahrens alle für derartige Umsetzungen üblichen organischen Solventien in Betracht. Vorzugsweise verwendbar sind aliphatische, alicyclische und aromatische Kohlenwasserstoffe, wie Pentan, Hexan, Petrolether, Cyclohexan, Benzol, Toluol oder Xylol, und außerdem Ether, wie Diethylether, Methyl-tert.-butyl-ether, Furan oder Dioxan, und weiterhin auch Pyridin.

Als Katalysatoren kommen bei der Herstellung der optisch aktiven 2-Aryl-propansäureamide der Formel (IV) alle für derartige Umsetzungen üblichen Reaktionsbeschleuniger in Frage. Vorzugsweise verwendbar sind tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können auch bei der Herstellung der optisch aktiven 2-Aryl-propansäureamide der Formel (IV) innerhalb eines größeren Bereiches variiert werden. Im allgemeinen arbeitet man bei Temperaturen zwischen -20°C und +120°C, vorzugsweise zwischen -10°C und +100°C.

Bei der Herstellung der optisch aktiven 2-Aryl-propansäureamide der Formel (IV) arbeitet man im allgemeinen unter Atmosphärendruck. Es ist aber auch möglich, unter erhöhtem Druck zu arbeiten.

Bei der Herstellung der optisch aktiven 2-Aryl-propansäureamide der Formel (IV) setzt man das jeweilige Säurechlorid mit einem Überschuß an Ammoniak bzw. wäßriger Ammoniak-Lösung, gegebenenfalls in Gegenwart einer geringen Menge an Katalysator, um. Die Aufarbeitung erfolgt nach üblichen Methoden. Im allgemeinen geht man so vor, daß man das Reaktionsgemisch, gegebenenfalls nach vorherigem Einengen, mit einem mit Wasser nur wenig mischbaren organischen Lösungsmittel extrahiert, die vereinigten organischen Phasen trocknet und unter vermindertem Druck einengt. Gegebenenfalls kann eine weitere Reinigung, zum Beispiel durch Chromatographie, vorgenommen werden.

Als Hypohalogenite kommen bei der Durchführung der dritten Stufe des erfindungsgemäßen Verfahrens Natriumhypochlorit und Natriumhypobromit in Betracht. Im allgemeinen geht man so vor, daß man diese Stoffe frisch herstellt, indem man Chlor oder Brom mit wäßriger Alkalihydroxidlösung z.B. Natronlauge umsetzt und die weitere Reaktion unmittelbar anschließt.

Die Reaktionstemperaturen können bei der Durchführung der dritten Stufe des erfindungsgemäßen Verfahrens innerhalb eines bestimmten Bereiches variiert werden. Im allgemeinen arbeitet man bei Temperaturen zwischen 0°C und 120°C, vorzugsweise zwischen 20°C und 100°C.

Bei der Durchführung der dritten Stufe des erfindungsgemäßen Verfahrens arbeitet man im allgemeinen unter Atmosphärendruck. Es ist aber auch möglich, unter erhöhtem Druck zu arbeiten.

Bei der Durchführung der dritten Stufe des erfindungsgemäßen Verfahrens setzt man auf 1 mol an optisch aktivem 2-Aryl-propansäureamid der Formel (IV) im allgemeinen 1 bis 10 mol, vorzugsweise 1 bis 5 mol an Natriumhypohalogenit ein. Die Aufarbeitung erfolgt nach üblichen Methoden. Im allgemeinen verfährt man in der Weise, daß man das Reaktionsgemisch mit einem mit Wasser wenig mischbaren, organischen Lösungsmittel extrahiert, die vereinigten organischen Phasen trocknet und unter vermindertem Druck einengt. Gegebenenfalls kann eine weitere Reinigung der anfallenden Produkte, zum Beispiel durch Chromatographie oder Destillation, vorgenommen werden.

Das erfindungsgemäße Verfahren eignet sich sowohl zur Herstellung von (R)- als auch von (S)-1-Aryl-alkylaminen. Welches der beiden Enantiomeren entsteht, hängt von der Wahl des chiralen Hydrierkatalysators aus Übergangsmetallsalz und Bisphosphin ab. Bei Verwendung eines Katalysators mit Liganden in der (R)-Konfiguration bildet sich bei der Durchführung der ersten Stufe des erfindungsgemäßen Verfahrens eine 2-Aryl-propansäure, die am asymmetrisch substituierten Kohlenstoffatom die (R)-Konfiguration aufweist. Daraus erhält man das entsprechende (R)-1-Aryl-alkylamin. Andererseits sind (S)-Amine zugänglich, wenn im ersten Schritt des erfindungsgemäßen Verfahrens ein Katalysator mit (S)-Konfiguration des Liganden verwendet wird, weil zunächst eine (S)-2-Aryl-propansäure entsteht.

Die nach dem erfindungsgemäßen Verfahren herstellbaren optisch aktiven 1-Aryl-alkylamine der Formel (I) sind wertvolle Zwischenprodukte zur Herstellung von Pharmazeutika oder von Wirkstoffen mit insektiziden, fungiziden oder herbiziden Eigenschaften (vgl. EP-A 0 519 211, EP-A 0 453 137, EP-A 0 283 879, EP-A 0 264 217 und EP-A 0 341 475). So erhält man zum Beispiel die fungizid wirksame Verbindung der Formel 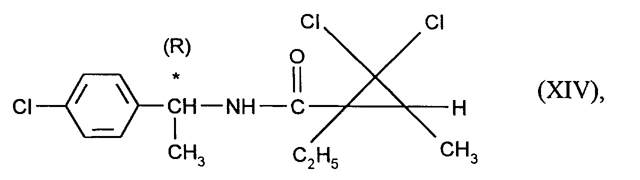 indem man das (R)-1-(4-Chlor-phenyl)-ethylamin der Formel 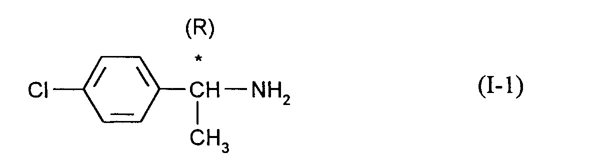 mit 2,2-Dichlor-1-ethyl-3-methyl-1-cyclopropancarbonsäurechlorid der Formel 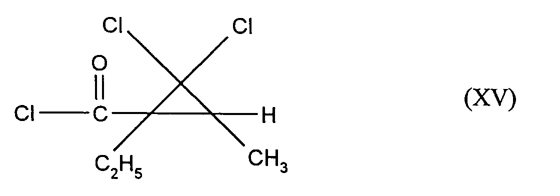 in Gegenwart eines Säurebindemittels und in Gegenwart eines inerten organischen Verdünnungsgmittels umsetzt.

Die Durchführung des erfindungsgemäßen Verfahrens wird durch die folgenden Beispiele veranschaulicht.

### Herstellungsbeispiele

### Beispiel 1

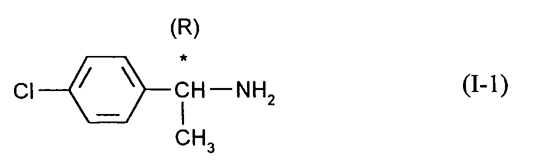

### 1. Stufe:

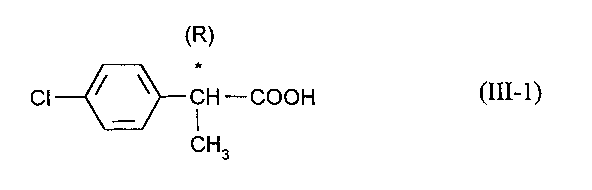

### Variante α:

Eine Lösung von 12,3 g 2-(4-Chlor-phenyl)-propensäure in 246 ml entgastem Methanol wird in einem Autoklaven unter Luftausschluß mit 370 mg [BINAP Ru (OAc)₂] versetzt. Anschließend wird Wasserstoff aufgepreßt und 72 Stunden bei Raumtemperatur unter einem Druck von 90 bar hydriert. Zur Aufarbeitung wird das Reaktionsgemisch zunächst unter vermindertem Druck eingeengt. Der verbleibende Rückstand wird in Essigsäureethylester aufgenommen. Das entstehende Gemisch wird zunächst mit 1N Salzsäure extrahiert und dann nacheinander mit wäßriger Natriumhydrogencarbonat-Lösung und mit wäßriger Natriumchlorid-Lösung ausgeschüttelt. Danach wird die organische Phase über Magnesiumsulfat getrocknet und unter vermindertem Druck eingeengt. Man erhält auf diese Weise 12,4 g (100 % der Theorie) an optisch aktiver 2-(4-Chlor-phenyl)-propansäure mit einem Enantiomerenüberschuß der (R)-Form von 90 %.

### Variante β:

Eine Lösung von 1,0 g 2-(4-Chlor-phenyl)-propensäure in einem Gemisch aus 10 ml entgastem Methanol, 10 ml entgastem Methylenchlorid und 0,84 ml Triethylamin wird in einem Autoklaven unter Luftausschluß mit 50,7 mg [(ClMeOBIPHEP)₂ Ru₂ Cl₄] versetzt. Anschließend wird Wasserstoff aufgepreßt und 72 Stunden bei Raumtemperatur unter einem Druck von 90 bar hydriert. Zur Aufarbeitung wird das Reaktionsgemisch zunächst unter vermindertem Druck eingeengt. Der verbleibende Rückstand wird in Essigsäureethylester aufgenommen.

Das entstehende Gemisch wird zunächst mit 1N Salzsäure extrahiert und dann nacheinander mit wäßriger Natriumhydrogencarbonat-Lösung und mit wäßriger Natriumchlorid-Lösung ausgeschüttelt. Danach wird die organische Phase über Magnesiumsulfat getrocknet und unter vermindertem Druck eingeengt. Man erhält auf diese Weise 1,0 g (100 % der Theorie) an optisch aktiver 2-(4-Chlor-phenyl)-propansäure mit einem Enantiomerenüberschuß der (R)-Form von 86,2 %.

### 2. Stufe:

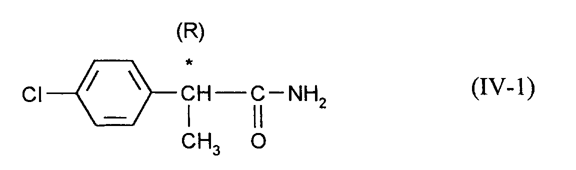

Eine Lösung von 10,6 g (0,057 mol) (R)-2-(4-Chlor-phenyl)-propansäure (ee-Wert 90,8%) in 200 ml Chloroform wird bei Raumtemperatur unter Rühren mit 18,1 g (0,152 mol) Thionylchlorid versetzt. Danach erhitzt man bis zur Beendigung der Gasentwicklung auf 65°C, (4-6 Stunden). Nach dem Abkühlen auf Raumtemperatur wird das Reaktionsgemisch unter vermindertem Druck eingeengt. Der verbleibende ölige Rückstand (14,1g) wird ohne zusätzliche Reinigung weiter umgesetzt, indem man 25 ml Methyl-tert.-butylether hinzufügt und die entstehende Lösung bei 0°C unter Rühren in 200 ml einer 25 %igen wäßrigen Ammoniak-Lösung gibt. Das Reaktionsgemisch wird 16 Stunden bei Raumtemperatur gerührt und dann dreimal mit Methylenchlorid extrahiert. Die vereinigten organischen Phasen werden über Natriumsulfat getrocknet und dann unter vermindertem Druck eingeengt. Man erhält auf diese Weise 9,38 g eines Produktes, das gemäß Gaschromatogramm zu 90% aus (R)-2-(4-Chlor-phenyl)-propionsäureamid (ee-Wert 90,7 %) besteht. Danach errechnet sich eine Ausbeute von 89 % der Theorie.

### 3. Stufe:

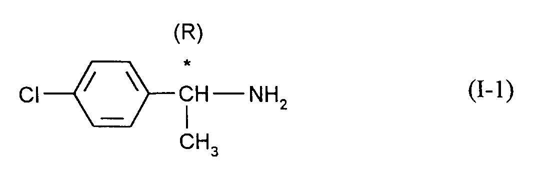

In eine Lösung von 9,4 g (0,235 mol) Natriumhydroxid in 75 ml Wasser werden bei 0°C unter Rühren 7,5 g (0,047 mol) Brom eingetropft. Das entstehende Gemisch wird auf -5°C gekühlt und unter Rühren mit 7,2 g (0,039mol) (R)-2-(4-Chlor-phenyl)-propionsäureamid (ee-Wert 90,7 %) versetzt. Anschließend wird 5 Stunden auf 50°C erhitzt. Nach dem Abkühlen auf Raumtemperatur extrahiert man das Reaktionsgemisch dreimal mit Methylenchlorid, trocknet die vereinigten organischen Phasen über Natriumsulfat und engt unter vermindertem Druck ein. Auf diese Weise erhält man ein Produkt, das gemäß Gaschromatogramm zu 83% aus (R)-1-(4-Chlor-phenyl)-ethylamin (ee-Wert 90,7%) besteht. Danach errechnet sich eine Ausbeute von 87 % der Theorie.

### Beispiel 2

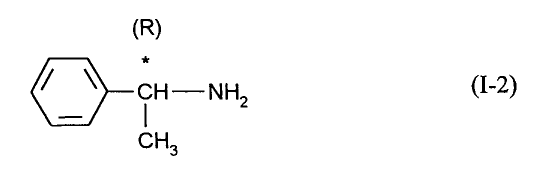

### 1. Stufe:

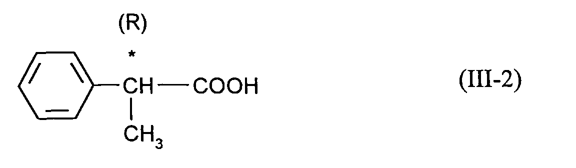

Eine Lösung von 1,0 g 2-Phenyl-propensäure in einem Gemisch aus 15 ml entgastem Methanol, 10 ml entgastem Methylenchlorid und 0,84 ml Triethylamin wird in einem Autoklaven unter Luftausschluß mit 50,7 mg [BIBFUP Ru cym I]I versetzt. Anschließend wird Wasserstoff aufgepreßt und 72 Stunden bei Raumtemperatur unter einem Druck von 90 bar hydriert. Zur Aufarbeitung wird das Reaktionsgemisch zunächst unter vermindertem Druck eingeengt. Der verbleibende Rückstand wird in Essigsäureethylester aufgenommen. Das entstehende Gemisch wird zunächst mit 1N Salzsäure extrahiert und dann nacheinander mit wäßriger Natriumhydrogencarbonat-Lösung und mit wäßriger Natriumchlorid-Lösung ausgeschüttelt. Danach wird die organische Phase über Magnesiumsulfat getrocknet und unter vermindertem Druck eingeengt. Man erhält auf diese Weise die optisch aktive 2-Phenyl-propansäure mit einem Enantiomerenüberschuß der (R)-Form von 87,3 %.

### 2. Stufe:

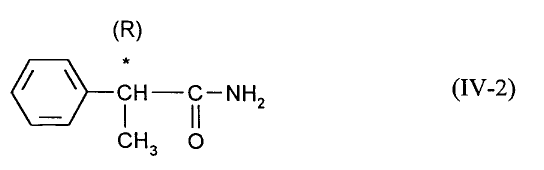

Eine Lösung von 10 g (0,067 mol) (R)-2-Phenyl-propansäure (ee-Wert >95 %) in Chloroform wird bei Raumtemperatur unter Rühren mit 11,9 g (0,1 mol) Thionylchlorid versetzt. Danach erhitzt man bis zur Beendigung der Gasentwicklung auf 40°C, (2 Stunden). Nach dem Abkühlen auf Raumtemperatur wird das Reaktionsgemisch unter vermindertem Druck eingeengt. Der verbleibende ölige Rückstand (11,3 g) wird ohne zusätzliche Reinigung weiter umgesetzt, indem man 50 ml Methyl-tert.-butylether hinzufügt und die entstehende Lösung bei 0°C unter Rühren in 500 ml einer 25 %igen wäßrigen Ammoniak-Lösung gibt. Das Reaktionsgemisch wird 16 Stunden bei Raumtemperatur gerührt und dann dreimal mit Methylenchlorid extrahiert. Die vereinigten organischen Phasen werden über Natriumsulfat getrocknet und dann unter vermindertem Druck eingeengt. Man erhält auf diese Weise 9,8 g eines Produktes, das gemäß Gaschromatogramm zu 93 % aus (R)-2-Phenyl-propionsaureamid besteht. Danach errechnet sich eine Ausbeute von 91 % der Theorie.

### 3. Stufe:

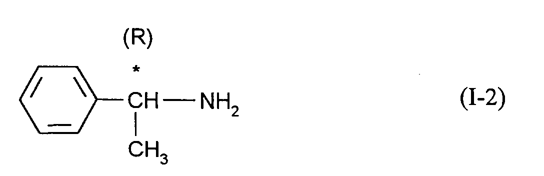

Ein Gemisch aus 6 g (0,04 mol) (R)-2-Phenyl-propionsäureamid, 4 g (0,1 mol) Natriumhydroxid und 30 ml Wasser wird bei Raumtemperatur unter Rühren mit 48 g (0,084 mol) Chlorlauge (Gehalt an aktivem Chlor: 12,5 %) versetzt. Anschließend wird 4 Stunden lang auf 80°C erhitzt. Nach dem Abkühlen auf Raumtemperatur extrahiert man das Reaktionsgemisch dreimal mit je 75 ml Methylenchlorid, trocknet die vereinigten organischen Phasen über Natriumsulfat und engt unter vermindertem Druck ein. Auf diese Weise erhält man 4,7 g eines Produktes, das gemäß Gaschromatogramm zu 85 % aus (R)-1-Phenyl-ethylamin (ee-Wert >95 %) besteht. Danach errechnet sich eine Ausbeute von 82.5 % der Theorie.

## Patentansprüche

1. Verfahren zur Herstellung von optisch aktiven 1-Aryl-alkylaminen der Formel 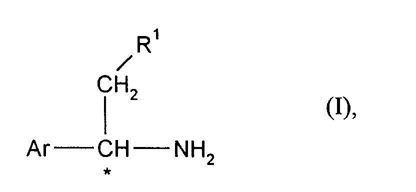 in welcher
R¹ für Wasserstoff oder Alkyl steht und
Ar für Phenyl oder Naphthyl steht, wobei jeder dieser Reste einfach bis fünffach, gleichartig oder verschieden substituiert sein kann durch Halogen, Alkyl, Alkoxy, Halogenalkyl, Halogenalkoxy, Cyano, Dialkylamino, Phenyl und/oder Benzyl,
dadurch gekennzeichnet, daß man
a) 2-Aryl-propensäuren der Formel 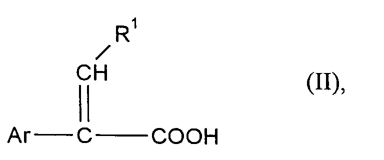 in welcher
R¹ und Ar die oben angegebenen Bedeutungen haben,
mit Wasserstoff in Gegenwart eines chiralen Komplexes aus einem Übergangsmetallsalz und einem chiralen Bisphosphin, sowie in Gegenwart eines Verdünnungsmittels und gegebenenfalls in Gegenwart eines tertiären Amins umsetzt,
b) die dabei entstehenden optisch aktiven 2-Aryl-propansäuren der Formel 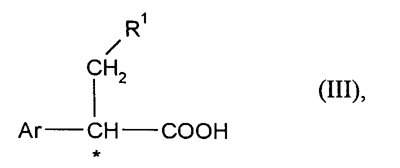 in welcher
Ar und R¹ die oben angegebenen Bedeutungen haben,
zunächst mit Thionylchlorid, gegebenenfalls in Gegenwart eines Verdünnungsmittels, und dann mit Ammoniak, gegebenenfalls in Gegenwart eines Verdünnungsmittels und gegebenenfalls in Gegenwart eines Katalysators, umsetzt und
c) die dabei erhaltenen optisch aktiven 2-Aryl-propansäureamide der Formel 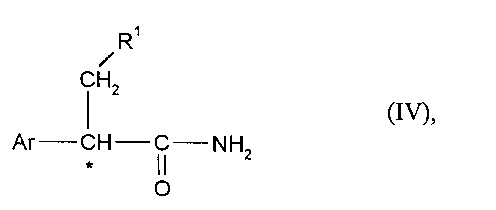 in welcher
R¹ und Ar die oben angegebenen Bedeutungen haben,
mit Natriumhypohalogeniten der Formel
Na-O-Hal (V),
in welcher
Hal für Chlor oder Brom steht,
in Gegenwart von Wasser umsetzt.

2. Verfahren gemäß Anspruch 1, dadurch gekennzeichnet, daß man als Ausgangsstoffe 2-Aryl-propensäuren der Formel (II) einsetzt, in denen
R¹ für Wasserstoff oder geradkettiges oder verzweigtes Alkyl mit 1 bis 6 Kohlenstoffatomen steht und
Ar für Phenyl oder Naphthyl steht, wobei jeder dieser Reste einfach bis dreifach, gleichartig oder verschieden substituiert sein kann durch Fluor, Chlor, Brom, geradkettiges oder verzweigtes Alkyl mit 1 bis 4 Kohlenstoffatomen, geradkettiges oder verzweigtes Alkoxy mit 1 bis 4 Kohlenstoffatomen, Halogenalkyl mit 1 bis 4 Kohlenstoffatomen und 1 bis 5 gleichen oder verschiedenen Halogenatomen, Halogenalkoxy mit 1 bis 4 Kohlenstoffatomen und 1 bis 5 gleichen oder verschiedenen Halogenatomen, Cyano, Dialkylamino mit 1 bis 4 Kohlenstoffatomen in jeder Alkylgruppe, Phenyl und/oder Benzyl.

3. Verfahren gemäß Anspruch 1, dadurch gekennzeichnet, daß man als Ausgangsstoffe 2-Aryl-propensäuren der Formel (II) einsetzt, in denen
R¹ für Wasserstoff, Methyl, Ethyl, n-Propyl, Isopropyl, n-Butyl, iso-Butyl, sek.-Butyl oder tert.-Butyl steht und
Ar für Phenyl oder Naphthyl steht, wobei jeder dieser beiden Reste einfach bis dreifach, gleichartig oder verschieden substituiert sein kann durch Fluor, Chlor, Brom, Methyl, Ethyl, n-Propyl, Isopropyl, n-Butyl, iso-Butyl, sek.-Butyl, tert.-Butyl, Methoxy, Ethoxy, Trichlormethyl, Trifluormethyl, Difluormethyl, Trifluormethoxy, Difluorchlormethoxy, Difluormethoxy, Cyano, Dimethylamino, Diethylamino, Phenyl und/oder Benzyl.

4. Verfahren gemäß Anspruch 1, dadurch gekennzeichnet, daß man bei der Durchführung der ersten Stufe Komplexe von Ruthenium-, Iridium- oder Rhodium-Salzen mit chiralen Bisphosphin-Liganden als Katalysatoren einsetzt.

5. Verfahren gemäß Anspruch 1, dadurch gekennzeichnet, daß man bei der Durchführung der ersten Stufe Komplexe von Ruthenium-Salzen mit chiralen Bisphosphin-Liganden der Formeln 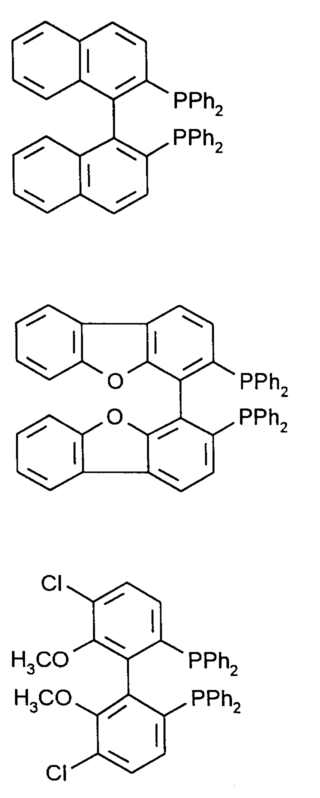 Ph = Phenyl
als Katalysatoren einsetzt.

6. Verfahren gemäß Anspruch 1, dadurch gekennzeichnet, daß man bei der Durchführung der ersten Stufe bei Temperaturen zwischen 5°C und 100°C arbeitet.

7. Verfahren gemäß Anspruch 1, dadurch gekennzeichnet, daß man bei der Durchführung der zweiten Stufe bei der Chlorierung bei Temperaturen zwischen -10°C und 100°C und bei der Amidherstellung bei Temperaturen zwischen -20°C und +120°C arbeitet.

8. Verfahren gemäß Anspruch 1, dadurch gekennzeichnet, daß man bei der Durchführung der dritten Stufe bei Temperaturen zwischen 0°C und 120°C arbeitet.
